# EUROPEAN PATENT APPLICATION

(11) **EP 2 100 595 A1**
(43) Date of publication of application: **16.09.2009**
(21) Application number: 08152550.3
(22) Date of filing: 10.03.2008
(51) Int. Cl.: A61K 9/00, A61K 9/16, A61K 9/20, A23L 1/00, A23L 1/22

(54) **Compressed tablets**

(71) Applicant: The Procter and Gamble Company, Cincinnati, Ohio 45202 (US)
(72) Inventor: Khanolkar, Jayant, Eknath, Surbiton Berkshire KT5 8RD (GB); Hallissey, Martin, Farnborough Hampshire GU14 6EB (GB); Kilpatrick, Lynn, Eileen, Campbell, Ascot Berkshire SL5 7JZ (GB)
(74) Representative: Clemo, Nicholas Graham

(57) **Abstract**

The invention provides a compressed tablet including a volatile active agent, preferably menthol, within a spray-dried granule further comprising a starch carrier and, optionally, a polyol granulating agent. The compressed tablet can be a two layer tablet comprising an effervescent layer and a non-effervescent layer with the spray-dried granule incorporated into the effervescent layer. The tablets herein arc useful for providing dccongcstant benefits.

The present invention further relates to the use of starch for increasing the rate of release of menthol from a compressed tablet. The use of starch as a carrier for the volatile active agent has been found to not only improve its rate of release from the tablet compared to other carriers, such as gum arabic, or compared to not using a carrier at all, but also to impart improved smoothness to the tablet when sucked.

## Description

### FIELD OF THE INVENTION

The present invention relates to compressed tablets that rapidly deliver a decongestant benefit to the nasal cavity.

### BACKGROUND OF THE INVENTION

Products in the form of boiled sweets or compressed tablets for delivery of respiratory benefits, such as allowing easier breathing through the clearing of nasal passages or soothing sore or irritated throats, are known. Such products typically include one or more active agents, which may include a decongestant, such as menthol. The active agent is released upon sucking or chewing the product.

It is desirable for such a product to provide fast release of the active agent. One way of controlling the release of active ingredients is through encapsulation. This is typically done in order to provide for delayed or prolonged release.

It has now surprisingly been found, at least in the context of a compressed tablet, that faster release of certain active agents can be provided by spray-drying them with starch. In certain embodiments the fast release may further be enhanced by building the spray-dried agent into an effervescent layer. This fast release can be coupled with prolonged release by combining the fast release active agent with a slow release active agent, the latter can be included within a separate, optionally non-effervescent, layer of a two layer tablet.

Compressed tablets including both effervescent layer and non-effervescent layers are known in the art. WO 2005/102066 discloses breath freshening confectionery products having at least one abrasive surface. The embodiment of Example 1 therein is a two layer tablet including two, non-effervescent layers each of which comprises menthol, peppermint and an encapsulated flavour. The embodiment of Example 2 of WO 2005/102066 is a two layer tablet including a first, non-effervescent, layer and a second, effervescent, layer. Each layer is flavoured with lemon/menthol. WO 2006/044595 discloses effervescent, pressed gum tablets for the delivery of active components to the oral cavity. Some of its embodiments are multi-layer tablets and the embodiment of its Example 3 is a two layer tablet comprising a non-effervescent gum layer and an effervescent tablet layer. Each layer comprises an undisclosed flavour. According to the general disclosure of WO 2006/044595, flavours for its tablets can be spray-dried or can alternatively be absorbed onto water soluble materials such as such as cellulose, starch, sugar, maltodextrin and gum arabic. Gum-like materials, especially as used in chewing gums can slow down or even withhold the release of volatile active agents.

### SUMMARY OF THE INVENTION

In one embodiment herein the invention provides a compressed tablet comprising a compressed, non-gum, powder base and a volatile active agent, wherein the volatile active agent is included within a spray-dried granule comprising:
a) the volatile active agent;
b) a carrier; and
c) optionally, a polyol granulating agent;
characterised in that the carrier is starch.

In one embodiment the compressed tablet is a two layer tablet comprising an effervescent layer and a non-effervescent layer and the spray-dried granule is incorporated into the effervescent layer.

In some embodiments the release of the active agent may further be improved by including a polyol granulating agent in the spray-dried granule.

The tablets herein are useful for providing decongestant benefits.

The present invention further relates to the use of starch for increasing the rate of release of menthol from a compressed tablet.

The use of starch as a carrier for the volatile active agent has been found to not only improve its rate of release from the tablet compared to other carriers, such as gum arabic, or compared to not using a carrier at all, but also to impart improved smoothness to the tablet when sucked.

### DETAILED DESCRIPTION OF THE INVENTION

All percentages and ratios herein are by weight unless specified otherwise.

As used herein, the term "compressed tablet" means a solid product formed by the compression of a powder base such that it remains in unitary form whilst being delivered to and handled by an end consumer. The compressed tablets herein are non-gums, i.e. they do not contain sufficient gum base materials, such as elastomers and the like, as understood in the art, to impart a chewable character to the tablet. The tablets are preferably absent or essentially absent elastomeric gum base materials.

As used herein, the term "volatile active agent" refers to a material having a sufficient vapour pressure at body temperature that, when released into the oral cavity, it is essentially immediately sensorially detectable by a user in the nasal cavity and so is capable of providing a breathing benefit by, for example, a decongestant action, or of providing relief to irritated nasal passages. Preferred volatile active agents have a boiling point in the range from 120°C to 300°C at atmospheric pressure. More preferred volatile active agents are menthol, eucalyptol and camphor. Menthol is especially preferred. Other cooling agents such as carboxamides can also be useful. Menthol can be used in essentially pure form or as part of a more complex essential oil, especially peppermint oil. Likewise, eucalyptol can be provided by eucalyptus oil. Levels of the volatile active agent are typically from 0.1% to 5%, preferably from 0.5% to 2%, by weight of the total tablet.

### Compressed powder base

The compressed tablet herein is made, using methods known in the art, by direct compression of a powder base. Where the tablet has more than one layer, the powder base for each layer can be the same or different. Powder bases for the tablet or each of the layers of the tablet can comprise a bulk powder selected from the group consisting of sugar powders, non-sugar polyol powders, such as sorbitol, mannitol and xylitol, and combinations thereof. The bulk powders can be crystalline or non-crystalline. A preferred bulk powder for each tablet layer herein is sorbitol. The powders are preferably sieved to a maximum particle size of less than 250 µm.

### Spray-dried granules

The compressed tablets herein further include a spray-dried granule which includes the volatile active agent and a starch carrier. The starch can be a modified starch. Many different grades of starch are commercially available and are suitable for spray-drying. The overall level or 'loading' of the volatile active agent within the spray-dried granule is typically from 10% to 40%, preferably from 20% to 35%, by weight of the total granule. In certain embodiments the spray-dried granule further includes a polyol granulating agent. The polyol granulating agent may improve the subsequent processing of the spray-dried granule, such as its compression into the tablet and may also improve the rate of release of the volatile active agent from the granule. Preferred polyol granulating agents are sugar alcohols such as sorbitol, mannitol and xylitol, especially sorbitol. When used, the polyol granulating agent may be included at a level of from 3% to 30%, preferably from 10% to 25%, by weight of the spray-dried granule. The spray-dried granules can be prepared by methods known in the art and include forming a solution or slurry of the volatile active agent, the starch carrier and, optionally the polyol granulating agent in an aqueous, ethanolic, or aqueous ethanolic medium and spray-drying.

### Other ingredients

The compressed tablets herein may further include amounts of conventional additives selected from the group consisting of high intensity sweeteners, tableting aids, nutrient minerals, flavours, coloring agents, antioxidants, medicaments, and mixtures thereof.

Useful high-intensity sweeteners include:
a) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
b) dipeptide based sweeteners, such as L-aspartic acid derived sweeteners, such as L-aspartyl-L-phenylalanine methyl ester (Aspartame), L-alpha-aspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, and mixtures thereof;
c) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, such as Sucralose; and
d) protein based sweeteners such as thaumaoccous danielli (Thaumatin I and II).

The intense sweetener may be present in amounts from 0.001% to 3%, by weight of the total tablet, depending upon the sweetener or combination of sweeteners used.

In some embodiments, it may be desirable to combine a food-grade tableting aid with the powder base. Tableting aids assist in processing the powder base into pressed tablets and are also used to prevent excess wear on dies and punches in tableting manufacture. Examples of suitable food-grade tableting aids include: metallic stearates; fatty acids; hydrogenated vegetable oil; partially hydrogenated vegetable oils; animal fats; polyethylene glycols; polyoxyethylene monostearate; talc; silicon dioxide; and combinations thereof. Tableting aids may be present in amounts of about 0-6% by weight of the tablet or tablet layer. A preferred tableting aid herein is magnesium stearate.

Nutrient minerals may include salts such as calcium and zinc salts. Medicaments may include other, perhaps non-volatile, decongestants and/or analgesics. Medicaments may also include antibacterial agents such as chlorhexidine, cetyl pyridinium chloride, and anti-bacterial essential oils such as thymol and eugenol. The tablets may also include an anti-oxidant such as Vitamin C.

### Effervescent System

Compressed tablets according to one embodiment of the present invention also include an effervescent system. The effervescent system includes an edible acid and a base, which react upon solubilisation with saliva to generate effervescence.

The base may be selected from, but is not limited to, alkali metal carbonates, alkali metal bicarbonates, alkaline earth metal carbonates, alkaline earth metal bicarbonates and combinations thereof. The edible acid may be selected from, but is not limited to, citric acid, phosphoric acid, tartaric acid, malic acid, ascorbic acid or combinations thereof.

In some embodiments, the edible acid and/or base may be encapsulated. Encapsulation may be desirable because it lends further moisture-resistance to the components, thereby preventing premature reaction of the acid and base components. The acid and base may be encapsulated as microcapsules or micro-particles as described in WO 2004/064544. Suitable encapsulants include, but are not limited to, fats, polymers, carbohydrates and combinations thereof.

The effervescent system may be present in amounts of from 3% to 30%, preferably from 10% to 20% by weight of the effervescent layer.

In addition to the volatile active agent, the tablets of the invention may further include other flavour agents known to the skilled person, such as natural and artificial flavours. These flavourings may be chosen from synthetic flavour oils and flavouring aromatics and/or oils, oleoresins and extracts derived from plants, leaves, flowers, fruits, and so forth, and combinations thereof. Non-limiting representative flavour oils include spearmint oil, cinnamon oil, oil of wintergreen (methyl salicylate), clove oil, bay oil, anise oil, thyme oil, cedar leaf oil, oil of nutmeg, allspice, oil of sage, mace, oil of bitter almonds, and cassia oil. Other useful flavourings are artificial, natural and synthetic fruit flavours such as vanilla, and citrus oils including lemon, orange, lime, grapefruit, and fruit essences including apple, pear, peach, grape, strawberry, raspberry, cherry, plum, pineapple, apricot and so forth. These flavouring agents may be used in liquid or solid form and may be used individually or in admixture. The flavour agent may be employed in either liquid form and/or dried form. When employed in the latter form, suitable drying means such as spray drying the oil may be used. Alternatively, the flavour agent may be absorbed onto water soluble materials, such as cellulose, starch, sugar, maltodextrin, gum arabic and so forth or may be encapsulated.

Techniques for preparing pressed tablets according to the invention are known to the person skilled in the art and are described in greater detail in e.g., WO 2005/102066.

### EXAMPLES

### Example 1

The following example is a two layer, compressed tablet according to a first embodiment of the invention. The tablet is manufactured to a total weight of about 1.6 g, of which the first, non-effervescent layer comprises about 80% of the total and the second, effervescent layer comprises the remaining 20% of the total table weight. In the table below, individual ingredient levels are expressed as a percentage of the respective layer weight.

| **1st Layer** | % w/w |
|---|---|
| | |
| Sorbitol | 97.575 |
| Peppermint oil | 0.500 |
| Food colouring | 0.030 |
| Menthol, spray dried with modified maize starch¹ | 1.000 |
| Acesulfame K | 0.045 |
| Aspartame | 0.050 |
| Magnesium stearate | 0.800 |
| Total | 100.000 |
| | |

| **2nd Layer** | % w/w |
|---|---|
| | |
| Sorbitol | 82.735 |
| Lemon oil | 0.150 |
| Menthol, spray dried with modified maize starch to form granules¹ | 1.000 |
| Acesulfame K | 0.045 |
| Aspartame | 0.070 |
| Sodium hydrogen carbonate | 8.750 |
| Citric acid, anhydrous | 6.251 |
| Magnesium stearate | 1.000 |
| Total | 100.000 |

| | |
|---|---|
| ¹ The loading of the menthol in the total starch/menthol mixture is a maximum of 50% and typically about 30%. | |

A flavour oil pre-mix for the first layer is made by mixing the peppermint oil with a portion (∼25% of the layer total) of the sorbitol and the sweeteners. Similarly, a colour pre-mix for the first layer is made by mixing the colorant (a food-grade blue lake) with a further portion (10 - 15% of the layer total) of the sorbitol. Each pre-mix is sieved through a 250 µm sieve and thoroughly mixed with the remainder of the sorbitol, the spray-dried menthol granules and the magnesium stearate to form a first powder blend which is compressed into first tablet layers under 2 kN of compression using standard tableting equipment. The second layer is similarly made from a second powder blend which is prepared by mixing a sieved pre-mix, comprising the lemon oil, the sweeteners and about 25% of the sorbitol, with the remainder of the sorbitol, the spray-dried menthol, the magnesium stearate and the effervescent system, comprising the citric acid and the sodium hydrogen carbonate. The second powder blend is used to form the second layer of the compressed tablet by compressing it on top of the compressed first layer under a pressure of ∼ 20 kN.

### Example 2

The following example is a single layer, compressed tablet according to a second embodiment of the invention. The tablet is manufactured to a total weight of about 1.6 g. In the table below, individual ingredient levels are expressed as a percentage of the total tablet weight.

| Ingredient | % w/w |
|---|---|
| Sorbitol | 97.575 |
| Peppermint oil | 0.500 |
| Food colouring | 0.030% |
| Menthol, spray dried with modified maize starch and sorbitol to form granules¹ | 1.000 |
| Acesulfame K | 0.045 |
| Aspartame | 0.050 |
| Magnesium stearate | 0.800 |
| Total | 100.000 |

| | |
|---|---|
| ¹ The loading of the menthol in the total starch/menthol/sorbitol mixture is 30%. | |

The tablet is made in essentially the same way as the 1^{st} layer of Example 1, using flavour and colour pre-mixes, except that the tablet is compressed under a pressure of ∼ 20 kN. The use of starch to spray-dry the menthol results in faster menthol release into the nasal cavity, as indicated by mass spectrometric analysis of the vapour sampled from a nose of a user sucking the tablet, than either using unencapsulated menthol or by using menthol encapsulated with gum arabic.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A compressed tablet comprising a compressed, non-gum, powder base and a volatile active agent, wherein the volatile active agent is included within a spray-dried granule comprising:
a) the volatile active agent;
b) a carrier; and
c) optionally, a polyol granulating agent;
**characterised in that** the carrier is starch.

2. A compressed tablet according to Claim 1 wherein the polyol granulating agent is present and is selected from sugar alcohols.

3. A compressed tablet according to Claim 2 wherein the polyol granulating agent is sorbitol.

4. A compressed tablet according to any preceding claim wherein the polyol granulating agent is present at a level of from 3% to 30% by weight of the spray-dried granule.

5. A compressed tablet according to any preceding claim which is a two-layer tablet comprising:
a) a first, non-effervescent, layer comprising
a first compressed powder base; and
a volatile active agent; and
b) a second, effervescent, layer comprising:
a second compressed powder base;
an effervescent system; and
the spray-dried granule.

6. A compressed tablet according to any preceding claim wherein each compressed powder bases is selected from the group consisting of sugar powders, non-sugar polyol powders and combinations thereof.

7. A compressed tablet according to Claim 6, wherein each compressed powder base is sorbitol.

8. A compressed tablet according to any preceding claim, wherein the volatile active agent is selected from menthol, eucalyptol and camphor.

9. A tablet according to any preceding claim for providing a decongestion benefit to a user.

10. The use of starch for increasing the rate of release of menthol from a compressed tablet comprising a non-gum, compressed powder base.
